# EUROPEAN PATENT APPLICATION

(11) **EP 2 856 896 A1**
(43) Date of publication of application: **08.04.2015**
(21) Application number: 14185860.5
(22) Date of filing: 22.09.2014
(51) Int. Cl.: A43B 1/00, A43B 13/12, A01N 25/10, A01N 25/34, A01N 63/00, C08K 5/00, C12N 1/20, C12N 3/00, D01F 1/10, A63B 21/00

(54) **Impregnated odour control products and methods of making the same**

(30) Priority: 23.09.2013 CA 2828174; 23.09.2013 US 201314034109
(71) Applicant: Life Science TGO, SRL, Bridgetown (BB)
(72) Inventor: Chow, Phillip, P, Markham, Ontario L3R 0G2 (CA); Ciupa, Catherine, Toronto, Ontario M8W 1R8 (CA)
(74) Representative: Gowshall, Jonathan Vallance

(57) **Abstract**

A product capable of controlling odours arising from organic matter, and a method of making the product. The product comprises an admixture of a moldable thermoplastic and benign dormant bacteria in a sporulated form, which admixture is formed by mixing the benign dormant bacteria into the moldable thermoplastic when the moldable thermoplastic is melted, and molding the admixture into a molded product. The benign dormant bacteria are embedded throughout the molded product, including being buried within a body of the molded product and being presented on a surface of the molded product. The benign dormant bacteria presented on the surface of the molded product are activatable upon exposure of the surface of the molded product to the organic matter, and the benign activated bacteria digest the organic matter to control production of offensive odours. Abrasive wear of the surface of the molded product exposes fresh benign dormant bacteria buried in the body to the surface for additional odour control.

## Description

### FIELD OF THE INVENTION

The present invention relates to the field of materials which are capable of controlling odour arising from certain organic matter. More particularly, the present invention relates to materials configured to control odours by using benign bacterial agents to consume and digest the organic matter which can otherwise lead to bad odours and to a method of manufacturing such odour control materials. The effect of the odour control materials is long lasting.

### BACKGROUND OF THE INVENTION

There are many instances where owing to deposits of organic matter on surfaces, offensive odours can arise through the presence of the organic matter and its decomposition. In fact, many materials utilized in household applications are susceptible to soiling by organic based material, which if allowed to remain, can give rise to malodours.

Deposits of organic materials on surfaces may also give rise to other concerns. Many of the organic materials, such as bodily fluids like sweat, blood and saliva, organic waste such as feces and urine, spilt food such as syrup and eggs, and beverages such as milk, wine and soft drinks etc., are capable of supporting bacterial growth. Some of the bacteria that may grow as a result of a deposit of organic material may give rise to odour due to decomposition and incomplete digestion of the organic material. Some of the bacteria may also have the potential of causing disease in persons exposed to them.

In order to control/reduce odours, there have been attempts to reduce the number of bacteria present on surfaces by utilizing various anti-microbial agents. See for example U.S. Pat. Nos. 4,110,504, and 5,024,840, which describe applying anti-microbial agents to carpet in a manner similar to the way stain blockers are applied to carpet. However, the use of anti-microbials raises concerns such as the potential that some of the bacteria may become resistant to effects of the anti-microbials, and the release of such anti-microbials into the environment may have a detrimental effect on the environment.

Many bacterial and fungal genera are known for use in odour control due to their capability for producing enzymes that are capable of breaking down organic material. Several such bacterial genera such as *Bacillus, Lactobacillus, Enterobacter, Streptococcus, Nitrosomonas, Nitrobacter, Pseudomonas, Alcaligens and Klebsiella* amongst others are known for use in such applications, with *Bacillus and Lactobacillus sp.* being the most prevalent in use in various applications.

For example, European Patent Application No. 732,396 describes use of *Bacillus* sp. for odour control of feedstuffs used in farming and JP Patent Application No. 7-031,668 describes its use for odour control of toilets, shoe boxes and pet litter. Other uses of *Bacillus* for odour control for baby diapers and wallpaper are described in JP Patent Application Nos. 2-121,665 and 3-3-059,199 respectively. Preparations of sporulated *Bacillus* in a form for spraying or otherwise distributing on a deposit, especially of pet urine and feces, on a carpet for controlling odour are presently marketed by The Bramton Company of Dallas, Tex. under the trademark OUTRIGHT. Once the deposit is deodorized, the bacteria are depleted from the site or disposed of along with the deodorized material. In the event of a new deposit of organic material on the carpet, the treatment must be repeated.

U.S. Pat. Nos. 6,974,691, and 7,314,748 disclose methods for controlling odour associated with spills of organic material which involve applying to surfaces, including, carpet, plastic and wood, a preparation of dormant bacteria, which when activated are effective to control odours. The dormant bacterial preparation is adhered to the surface, such that when the treated surface is exposed to organic material which can cause odours, the dormant bacteria are capable of becoming active and digest the organic material.

Although, the methods described in the U.S. '691 and '748 patents are effective for surface treating fibrous materials, including carpet, batting used for mattresses, pillows and pads, as well as other relatively porous surfaces encountered in the household or commercial environment, semiporous surfaces such as wood and nonporous surfaces such as plastic, they are not effective in treating non-fibrous materials that are subjected to surface abrasion. Therefore, there remains a need to counteract the effects of deposits of organic material, and especially for controlling odour associated with the deposits, including in non-fibrous materials such as thermoplastics, where the effects of the odour control are preventative and long lasting, even in high wear applications.

Other prior art patent documents of general interest include: Canadian Pat. Document Nos. 2,243,011, and 2,369,469; U.S. Pat. Nos. 3,636,927, 3,720,606, 3,720,606, 3,892,846, 3,941,090, 4,465,019, 4,607,594, 4,680,212, 4,704,989, 4,839,212, 4,844,010, 4,925,707, 4,946,672, 5,154,594, 5,683,575, 5,741,553, 5,863,882, 6,265,191, 6,325,934, 6,967,025, 7,507,402, 7,795,119; U.S. Pat. App. Pub. Nos. 2003/0003138, 2003/0012810, 2003/0089381, 2003/0126688, 2008/0075794, and 2012/0114579; PCT Int'l Pat. App. Pub. Nos. 96/19611, 97/25865, 97/43385, 99/46350, 00/03752, 00/63338, 02/33035, 03/056096, 03/064755; European Pat. App. Pub. Nos. 0039522, 0076447, 0476915, 0878202, and 1096959; U.K. Pat. App. Pub. No. 2362814; Japanese Pat. Document Nos. 5-153971, 7-222790, and 9-28377; Czechoslovakian Pat. Document No. CS246119; Soviet Union Pat. Document No. SU1091889.

Other non-patent documents of general interest include:
- Brochure for BI-CHEM.RTM. BIOCLEAN Carpet Cleaner;
- Brochure for BI-CHEM.RTM. GC 600L;
- Brochure for BI-CHEM.RTM. MSB 4X;
- Chemical Abstracts, vol. 108, No. 19, May 1998, Abstract No. 169120y; XP002122802;
- CHEMICAL ABSTRACTS, vol. 108, no. 20, May 1998 (1998-05) Columbus, Ohio, US; abstract no. 169120y, XP002122802 & CS 246 119 A (DOSTAL JAROSLAV; VIEST MIROSLAV) 16 October 1986 (1986-10-16);
- DATABASE WPI Section Ch, Week 199715 Derwent Publications Ltd., London, GB; Class A97, AN 1997-159089 XP002122803 & JP 09 028377 A (AZUMA K), 4 February 1997 (1997-02-04);
- Database WPI, Week 199329, Derwent Publications Ltd., London, GB; An 1993-231489; XP002210738 Deodorise Supress Excretion Bad Smell Contain Bacillus Magaterium Effect Range Malodorous
- 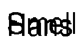 ;G. Schegel et al., General Microbiology, 7.sup.th ed., p. 83 (1993);
- Response to Summons in Opposition by Novozymes Biologicals, Inc. in European Patent No. 1 096 959 B1 with attached Declaration and Exhibits (139 pages) (Aug. 2005); and
- XP002210738 Deodorise Supress Excretion Bad Smell Contain Bacillus megaterium Effect Range Malodorous Smell.

### SUMMARY OF THE INVENTION

The present invention is directed to an improved product capable of controlling odours arising from organic matter, and methods of making the product.

Preferably, the product is a molded (i.e. compression molded, injection molded, blow molded, extrusion molded, etc.) Ethylene Vinyl Acetate (EVA) thermoplastic, having benign dormant bacteria embedded generally throughout, buried within, and presented on the surface thereof. The benign dormant bacteria presented on the surface of the molded product are activatable, upon exposure to organic matter, to generate enzymes and digest the organic matter while controlling the production of offensive odours. Abrasive wear of the surface of the molded product exposes fresh benign dormant bacteria buried within the molded product to the surface of the product to provide renewed and additional odour control.

Examples of molded products include automotive products such as car seats, child safety seats, carpet underlay, and headliners; marine products such as life jackets; electronics accessory products such as work station mats and mouse pads; sport and leisure products such as sports mats, gym mats, exercise mats, yoga mats, garden mats, personal protective equipment, sport protective pads, toys and games, camping bedrolls, backpacks, hand shopping baskets, luggage, travel bags and visors; footwear such as shoes, clogs, flip flops, slippers, insoles and boots; and toys such as play mats and blocks, etc.

According to an embodiment of the present invention the molded product is formed into fibers, pellets, sheets, blocks or tiles.

According to another embodiment of the present invention the sheet, block, tile form of the molded product is formed into a secondary structure by abrading, shaping, forming or additional molding.

According to another embodiment of the present invention the fiber form of the molded product is formed into a secondary structure by braiding, twisting, weaving, knitting, crocheting, knotting, spinning, crimping or felting.

According to yet another embodiment of the present invention the secondary structure is formed into threads, yarns or textiles.

Examples of textiles include floor coverings, bedding, drapery, upholstery, a towel, at least a portion of an article of clothing, and at least a portion of a footwear.

Examples of floor coverings include carpets, carpet tiles, mats and rugs.

Examples of articles of clothing include dresses, coats, socks, pants, shirts, jackets and headwear.

Therefore, in accordance with one aspect of the present invention, there is provided a product capable of controlling odours arising from organic matter, said product comprising:
an admixture of a moldable thermoplastic and benign dormant bacteria in a sporulated form, formed by admixing said benign dormant bacteria and said moldable thermoplastic when said moldable thermoplastic is melted, said admixture being molded into a molded product;
said benign dormant bacteria being embedded throughout said product, including being buried within a body of said molded product and presented on a surface of said molded product, said benign dormant bacteria presented on said surface being activatable upon exposure of said surface to said organic matter;
wherein said benign activated bacteria digest said organic matter to control production of offensive odours, and abrasive wear of said surface exposes fresh benign dormant bacteria buried within said body to said surface for additional odour control.

In accordance with another aspect of the present invention, there is provided a secondary structure formed by cutting, abrading, shaping, forming, or additional molding, a sheet, block, or tile form of the above product.

In accordance with another aspect of the present invention, there is provided a secondary structure formed by braiding, twisting, weaving, knitting, crocheting, knotting, spinning, crimping, or felting, a fiber form of the above product.

In accordance with yet another aspect of the present invention, there is provided a method of making a product capable of controlling odours arising from organic matter, said method comprising the steps of:
a) melt-blending at a bacteria compatible temperature range:
   i) a moldable thermoplastic; and
   ii) benign dormant bacteria in a sporulated form; and
b) forming the melt-blended product of step (a) into a molded product, with said benign dormant bacteria being embedded generally throughout said molded product, including being buried within a body of said molded product and being presented on a surface of said molded product, said benign dormant bacteria presented on said surface being activatable upon exposure of said surface to said organic matter;
wherein said benign activated bacteria digest said organic matter to control production of offensive odours, and abrasive wear of said surface of said molded product exposes fresh benign dormant bacteria buried within body to said surface for additional odour control.

In accordance with yet another aspect of the present invention, there is provided a product capable of controlling odours arising from organic matter, said product being formed by the above method.

In accordance with yet another aspect of the present invention, there is provided a use of benign dormant bacteria in a sporulated form in the preparation of a product capable of controlling odours arising from organic matter, said product comprising an admixture of a moldable thermoplastic and benign dormant bacteria in a sporulated form, formed by admixing said benign dormant bacteria and said moldable thermoplastic when said moldable thermoplastic is melted, said admixture being molded into a molded product; said benign dormant bacteria being embedded throughout said molded product, including being buried within a body of said molded product and being presented on a surface of said molded product, said benign dormant bacteria presented on said surface being activatable upon exposure of said surface to said organic matter; wherein said benign activated bacteria digest said organic matter to control production of offensive odours, and abrasive wear of said surface exposes fresh benign dormant bacteria buried within said body to said surface for additional odour control.

### BRIEF DESCRIPTION OF THE DRAWINGS

Reference will now be made to the preferred embodiments of the present invention with reference, by way of example only, to the following drawings in which:
Figure 1 is a product according to one embodiment of the present invention;
Figure 2 are results of tests of bacterial growth in test specimens of product of Figure 1;
Figure 3 is a footbed for a sandal according to another embodiment of the present invention;
Figure 4 is a sandal assembled using the footbed of Figure 3;
Figure 5 is a footbed for a sandal according to another embodiment of the present invention;
Figure 6 is a sandal assembled using the footbed of Figure 5; and

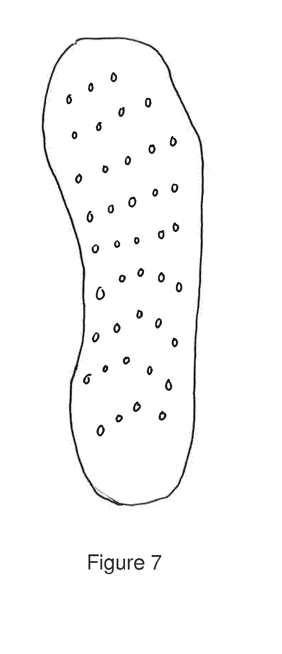

Figure 7 is an insole for a shoe according to another embodiment of the present invention.

### DETAILED DESCRIPTION OF THE PREFERRED EMBODIMENTS

The present invention is described in more detail with reference to exemplary embodiments thereof as shown in the appended drawings. While the present invention is described below including preferred embodiments, it should be understood that the present invention is not limited thereto. Those of ordinary skill in the art having access to the teachings herein will recognize additional implementations, modifications, and embodiments which are within the scope of the present invention as disclosed and claimed herein.

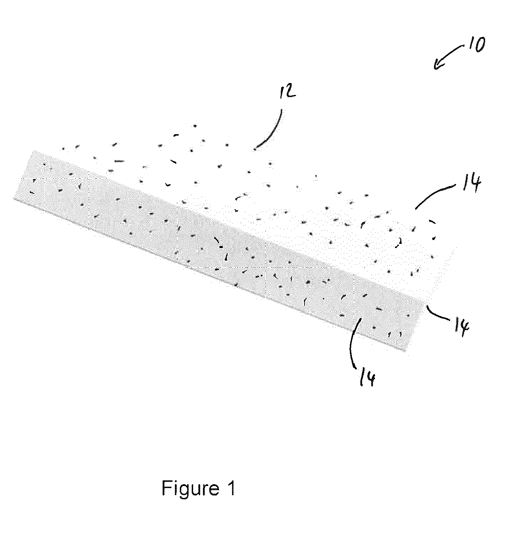

A product capable of controlling odours arising from organic matter is shown generally with reference numeral 10 in Figure 1, according to an embodiment of the present invention. As shown, the product 10 is an admixed combination of a moldable thermoplastic containing benign dormant bacteria 12 in a sporulated form, which combination was melt-blended and molded a molded product, in this case a sheet, from which the square piece of 10, shown in Figure 1, was cut. Preferably, the moldable thermoplastic is an ethylene vinyl acetate (EVA), however, as discussed in more detail below, different suitable moldable thermoplastics may be utilized as desired. The benign dormant bacteria 12 are embedded generally throughout the product 10, including being buried within the body of the product 10 as well as being presented on the surface 14 of the product 10. The benign dormant bacteria 12 presented on the surface 14 of the product 10 are activatable upon exposure of the surface 14 of the product 10 to organic matter. When activated, bacteria 12 digest the organic matter to control production of offensive odours.

Preferably, the product 10 contains a surface population of the benign dormant bacteria 12 large enough to digest the organic matter at a rate sufficient to control by reducing or eliminating odours associated with the organic matter. In this regard, good results have been obtained with between 10⁶ and 10⁸ of the benign dormant bacteria 12, estimated by calculating colony forming units (cfu), being present on the surface of the product 10, per square inch. Preferably, the surface population of the benign dormant bacteria 12 will be at least 10⁷ cfu per square inch.

Beneficially, abrasive wear of the surface 14 of the product 10 exposes fresh benign dormant bacteria buried in the body to the surface 14 for additional odour control. Accordingly, the product 10 contains a buried population of the benign dormant bacteria 12 large enough to continue to digest the organic matter at a rate sufficient to control odours associated with the organic matter as the surface wears. In this regard, good results have been obtained with between 10¹² and 10¹⁶ of the benign dormant bacteria 12 being present per cubic inch of the product 10.

The present invention comprehends adding an effective amount of benign dormant bacteria to reduce and thus control odours. While certain ranges identified it will be understood that different applications may have different insult levels typically, which can vary. For example, a shoe insole may have heavy daily load of sweat, whereas another application may not receive such continual insults. In all cases it is preferable to provide the beneficial bacteria are present on a surface initially when insults occur, and to remain present during and after wear due to being incorporated into the material. As such specific amounts of bacteria present are intended to be shown as examples and not as limitations per se.

The product 10 is preferably made by first melt-blending at a bacteria compatible temperature range a mixture comprising a moldable thermoplastic, then continuing to blend the moldable thermoplastic mixture in a double-roller blending or kneading machine, and adding a mixture of benign dormant bacteria in a sporulated form. Preferably, one or more of a foaming agent (i.e. 3 to 10% w/w), a cross-linking agent (i.e. 1 to 5% w/w), a lubricant (i.e. 0.5% to 2% w/w), and a filler, can be included in the moldable thermoplastic mixture depending on the application, as will be appreciated by persons skilled in the art. The ingredients may be melt-blended together all at once, or in separate stages. In generally, it has been found that up to 1% w/w of an aqueous mixture of benign dormant bacteria can be added to a moldable thermoplastic mixture comprising EVA. However, it is contemplated that lower or higher % w/w of aqueous mixture of benign dormant bacteria may be used, depending on the moldable thermoplastic being used, for example in the range of 0.01 % to 5%

The bacteria compatible temperature range is governed mostly by the temperature of the moldable thermoplastic being used, and temperatures of up to 175°C have yielded acceptable results. It is helpful that the bacteria compatible temperature range be sufficient to melt the moldable so that the mixture of benign dormant bacteria and other ingredients will homogeneously blend or knead, and an effective amount of the benign dormant bacteria will be present to control odours. It will be understood that the bacteria compatible temperature range does not exceed a temperature at which the sporulated bacteria are destroyed. While certain bacteria compatible temperature ranges are identified it will be understood that different applications will require different temperatures. As such the temperatures described herein are intended to be shown as examples only and not as limitations.

The melt-blended product is then transferred from the blending/kneading machine and formed by molding into the molded product, with the benign dormant bacteria 12 being embedded generally throughout the product 10, including being buried within the body of the product 10 and being presented on a surface 14 of the product 10. The benign dormant bacteria 12 presented on the surface 14 of the product 10 are activatable upon exposure of the surface 14 of the product 10 to the organic matter, and upon activation, the benign activated bacteria digest the organic matter to control production of offensive odours. As mentioned above, abrasive wear of the surface 14 of the product 10 preferably exposes fresh benign dormant bacteria 12 buried in the body to the surface 14 for additional odour control.

The moldable thermoplastic may be an open-cell foam, or a close-cell foam, which can be compression molded, injection molded, blow molded, or extrusion molded, according to techniques well known in the art. Although preferred thermoplastics are EVA, those persons skilled in the art will appreciate that other moldable thermoplastic materials may be used in place of EVA, such as for example a polyethylene, a polypropylene, a polyvinyl chloride, a polyamide, an elastane or a polystyrene. All such moldable thermoplastics may be used herein.

It is important that the moldable thermoplastic mixture, when combined with the mixture of benign dormant bacteria, can be molded into the specific desired for the product 10, and a sufficient amount of the benign dormant bacteria 14 survive the processing and molding steps to form the product 10.

The product 10 may be formed into numerous shapes and sizes for countless applications.

For example, the product 10 may be formed into a fiber by extruding the moldable thermoplastic mixture impregnated with the mixture of benign dormant bacteria into fibers or filaments. The present invention also contemplates forming a secondary structure by braiding, twisting, weaving, knitting, crocheting, knotting, spinning, crimping, or felting, the fiber form of product 10, using techniques which are well known in the art.

Alternately, the product 10 may be formed into a sheet, a block, or a tile, by compression molding, injection molding, blow molding, or extrusion molding, using techniques well known in the art.

For example, compression molding can be used to make insoles for shoes, as follows. After forming the melt-blended product containing the moldable thermoplastic mixture and the mixture of benign dormant bacteria, it is kneaded at room temperature (i.e. about 20 to 25°C) for about 10 minutes, and then rolled into sheets. The sheets are cut, compressed into large sheets at 170°C to 175°C, and a pressure of about 150 kg/cm² for about 10 to 20 minutes during which process cross-linking takes place and the product is foamed. It is then pressed into an insole mold at approximately 160°C for 15 minutes.

As another example, injection molding can be used to make one piece as follows. After forming the melt-blended product containing the moldable thermoplastic mixture and the mixture of benign dormant bacteria, it is to a temperature of 70°C and injected into a mold. The mold is then hot pressed in an oven at 160°C for 7 minutes.

Forming a secondary structure by cutting, abrading, shaping, forming, or additional molding, the sheet, block, or tile, using well known techniques, is also contemplated.

As can now be appreciated, the moldable thermoplastic mixture impregnated with the benign dormant bacteria, can be made in large batches, and formed into bricks that are then cut into sheets, or it can be extruded into sheets, in either case, the sheets can then be put into molds and cold or hot formed into shapes, such as, for example, insoles or sandal beds. Alternately, the moldable thermoplastic mixture impregnated with the benign dormant bacteria can be extruded, placed onto molds, or injected into molds, for shaping.

The following is a non-exhaustive list of the numerous applications contemplated for the product 10 according to various embodiments of the present invention:
- threads;
- yarns;
- textiles;
- floor coverings (i.e. carpets; carpet underlays; carpet tiles; mats including sports mats, gym mats, exercise mats, yoga mats, children's play mats, and garden mats; and rugs);
- bedding;
- drapery;
- upholstery;
- towels;
- articles of clothing or portions thereof (i.e. dresses, coats, socks, pants, shirts, headwear, and jackets);
- footwear or portions thereof (i.e. sandals, shoes, boots, slippers, flip flops, clogs, insoles, footbeds, and outsoles);
- body protective gear
- toys and games (i.e. play mats, blocks);
- automotive products (i.e. car seats, child safety seats, carpet underlay, and headliners);
- marine products (i.e. life jackets);
- electronics accessory products (i.e. work station mats, mouse pads);
- sport and leisure products (i.e. sports mats, gym mats, exercise mats, yoga mats, garden mats, personal protective equipment, sport protective pads, camping bedrolls, backpacks, hand shopping baskets, luggage and travel bags, and visors).

Many bacterial genera are known to produce enzymes that are capable of breaking down organic matter. Such bacteria are particularly useful where the organic matter, if allowed to remain, will give rise to malodours. Several such bacterial genera such as *Bacillus, Lactobacillus, Enterobacter, Streptococcus, Nitrosomonas, Nitrobacter, Pseudomonas, Alcaligens,* and *Klebsiella* amongst others are known for use in such applications, with *Bacillus* and *Lactobacillus* sp. being the most prevalent in use in various applications. Strains of bacteria from the genera *Bacillus* are useful in practicing the present invention. The preferred benign dormant bacteria 12 are those which when activated by the presence of organic matter, produce an enzyme to break down and assist with digesting the organic matter, creating only water and carbon dioxide as by-products. Once the organic matter has been digested the preferred benign dormant bacteria 12 return to a dormant state until organic matter is presented again and the cycle repeats.

Preferably, the mixture of benign dormant bacteria for use in the present invention includes one or more strains of *Bacillus megaterium, Bacillus subtilus, Bacillus Licheniformis, Bacillus Cerus, Bacillus, Alvei, Bacillus Coagulans, Bacillus Pumillus, Bacillus Simplex, Bacillus Brevis,* and *Bacillus Amyloliquefaciens,* which are available for example from Envera, LLC, whose address is 220 Garfield Ave., West Chester, Pennsylvania, 19380, U.S.A. More preferably, the strains of bacteria for use in the present invention are selected from *Bacillus megaterium, Bacillus subtilus,* and *Bacillus Licheniformis* species. Each of these species a) effectively express enzymes that break down particular types of organic matter, without producing odorous metabolites, b) are ubiquitous in nature, c) are non-hazardous to humans, and d) are included in the Domestic Substances List (DSL) in Canada, and are therefore eligible to be imported and used within Canada.

The selection of the strains of bacteria for use in the present invention may depend upon many factors. One such factor is the nature of the organic matter most commonly expected for the particular application. However, the most common forms of organic matter include bodily fluids, or organic waste. In most cases, to activate the benign dormant bacteria, the organic matter will need to contain carbon, nitrogen, and sufficient moisture content. Typically the bodily fluids include sweat, blood, saliva, and semen. Typical organic wastes include feces, urine, soil, beverages, such as, coffee, tea, milk, wine, and soft drinks, other food, such as, syrup, and eggs, and the like.

In an indoor environment, the nature of the deposits of organic matter may differ from those of an outdoor environment. Therefore, due consideration may need to be given to the environment where the product 10 will most be used. Depending upon the nature of the deposited organic matter, the mixture of benign dormant bacteria 12 may be selected to contain strains having enhanced activity against such materials. Another factor that may affect the nature of the deposit is the geographical location of the surface of the product. This factor would especially relate to the nature of deposits of out-doors soil and to the nature of food deposits. Different regions are to have different soil types and different regions may also have differences in the foods commonly consumed due to cultural and environmental factors. In addition, the temperature of the product will influence the activity of the bacteria. Depending on the strain selected the bacteria will tend to exhibit enhanced activity at higher temperatures. At lower ambient temperatures, more active strains may be desired.

The mixture of benign dormant bacteria will typically comprise of one or more strains selected from the species described above, and a carrier. When utilizing a mixture of more than one strain, each of the individual strains may comprise between 3% and 97% of the total of the benign dormant bacteria present in the mixture. These percentages are based on the total cell number. When mixtures of more than two strains are employed, each of the strains is most preferably present in an amount of from 10% to 60% of the total bacteria in the preparation. Particularly preferred mixtures of benign dormant bacteria for general use in almost all applications are as follows:

| | **% of Total Bacteria** | | |
|---|---|---|---|
| **Species** | **Range** | **Preferred Range** | **Most Preferred** |
| *Bacillus megaterium* | 3-97 | 5-15 | 10 |
| *Bacillus subtilus* | 3-97 | 10-50 | 30 |
| *Bacillus licheniformis* | 3-97 | 40-80 | 60 |

| | | | |
|---|---|---|---|
| * Total benign dormant bacteria in mixture equals 100% | | | |

In a preferred embodiment of the present invention an effective amount of a bacterial composition comprising one or more strains selected from the group consisting of *Bacillus megaterium, Bacillus subtilus, Bacillus Licheniformis, Bacillus Cerus, Bacillus, Alvei, Bacillus Coagulans, Bacillus Pumillus, Simplex, Bacillus Brevis,* and *Bacillus Amyloliquefaciens* are provided in a state in which the composition may be combined with a moldable thermoplastic material. The effective amount is a sufficient number of benign dormant bacteria 12 to provide a relatively uniform coverage on the surface of the product 10 such that when any portion of the surface 14 is exposed to deposit of an odour causing organic matter, the activated benign bacteria will undergo rapid growth and consume the odour causing organic matter. The factors that can affect the number of benign dormant bacteria 12 to be used relate in most part to the nature of the product 10. As mentioned above, however, for most products between about 10⁶ and 10⁸ benign dormant bacteria per square inch of the surface 14 of the product is most effective about 10⁷ cfu per square inch being most preferred.

The bacteria may be provided in an aqueous carrier or a dry powder carrier. The purpose of the carrier is to transport the benign dormant bacteria 12 to, and facilitate with dispersing the benign dormant bacteria 12 throughout, the melt-blended mixture of moldable thermoplastic during the kneading step. In this regard, the aqueous carrier is preferred over the dry powder carrier, because the dry powder carrier has been found to be susceptible to dispersing into the air when being applied to the kneading moldable thermoplastic mixture. This phenomenon is problematic because when portions of the benign dormant bacteria mixture are dispersed into the air concentrations of some components of the melt-blended product will vary, which may adversely affect characteristics of the resulting product 10.

Distilled water can be used as the aqueous carrier if the benign dormant bacteria mixture will be combined with the moldable thermoplastic mixture immediately. According to this embodiment, the benign dormant bacteria 12 are in a spray dried, dry powder form, and simply rehydrated in the water to form the benign dormant bacteria mixture on site, just before the benign dormant bacteria mixture is combined with the moldable thermoplastic mixture, as discussed above. Preferably, the aqueous benign dormant bacteria mixture contains between 0.0001% and 2% w/w of the benign dormant bacteria, with the remainder being distilled water. What is desired is for the aqueous benign dormant bacteria mixture to contain a sufficient amount of benign dormant bacteria to result in at least 10⁶ benign dormant bacteria being present at the surface of the product 10 per square inch, as mentioned above, without introducing too much water to the moldable thermoplastic mixture so as not to significantly alter the characteristics or quality of the resulting product.

However, if the benign dormant bacteria mixture will not be used immediately, the aqueous form of the benign dormant bacteria mixture will preferably also include:
- 0.1% to 10% w/w of a surfactant, such as sodium lauryl sulphate, glucopon, or sterol, to break up the benign dormant bacteria clumps and disperse them in the solution;
- 0.03% to 2% of a buffer, such as a phosphate based buffer system comprising a blend of potassium phosphate monobasic and potassium phosphate dibasic, to maintain a pH of 6.5 to 7.5; and
- 0.001% to 1% preservative, such as a biocide, to a) prevent the benign dormant bacteria from germinating, and b) prevent contamination of the benign dormant bacteria mixture with outside bacteria.

It is contemplated that the benign dormant bacteria mixture may be provided either a concentrated form, which will need to be diluted prior to being used, in a ready to use form, which may be directly applied to the moldable thermoplastic mixture at the appropriate stage in the manufacturing process.

The preferred dry powder carrier is calcium carbonate or sodium bicarbonate in a fine powder form. It has been found that the dry powder form of the benign dormant bacteria mixture does not require a preservative, since the lack of moisture both inhibits germination of the benign dormant bacteria, and inhibits growth of outside bacteria. Thus, the preferred dry powder form of the benign dormant bacteria mixture includes:
- 0.0001% to 2% w/w of the benign dormant bacteria in a spray dried, powder form; and
- the remainder being calcium carbonate, or sodium bicarbonate in a fine powder form.
-

### PREPARATION EXAMPLE

A test specimen of product 10 was prepared according to an embodiment of the present invention, as follows.
1. Add 4,086 g (25.58% w/w) of ethylene-vinyl acetate (EVA) resin, 4,540 g (28.42% w/w) of CaCO₃, 908 g (5.68% w/w) of natural rubber, 100 g (0.63% w/w) of zinc oxide (ZnO), 35 g (0.22% w/w) of zinc salt of stearic acid (ZnSt), 1,135 g (7.10% w/w) of polyethylene (PE), and 2,951 g (18.47% w/w) of polyolefin elasotomer (POE) to a mixing machine set to a temperature of between 115°C and 120°C. Mix the mixture for 5 minutes.
2. Add 120 g (0.75% w/w) of dicumyl peroxide (DCP) cross-linking agent, 450 g (2.82% w/w) of AC foaming/blowing agent, and 1,500 g (9.39% w/w) black color dye to the mixture. Continue mixing for another 5 minutes.
3. Transfer the mixture to a double-roller blending/kneading machine with 21 inch diameter rollers set to a temperature of 110°C. Knead the mixture for about 2 minutes.
4. Add 150 g (0.94% w/w) of an aqueous dormant bacteria mixture comprising about 2.12x10⁹ cfu/ml of *Bacillus megaterium, Bacillus subtilus,* and *Licheniformis* in a ratio of about 1:3:6 obtained from Envera LLC, West Chester, Pennsylvania, U.S.A, by slowly pouring onto the mixture as it is being blended/kneaded on the roller of the double-roller blending/kneading machine.
5. Knead the mixture for 3 to 6 minutes.
6. Transfer the mixture to a cutting machine to cut the mixture into thin sheets of 1.2 mm thickness.
7. Put stacks of thin sheets about 16 mm high into a mold measuring 150 cm x 75 cm x 1.6 cm. Insert the mold into a blowing machine set at 171°C and apply a pressure of 183 kg/cm² for 22 minutes.
8. Release the pressure and remove the blown EVA slabs, which become about 32 mm thick, from the blowing machine.
9. Set the blown EVA slabs aside for a minimum of 2 hours to allow them to cool and shrink to a stable size, resulting in smooth EVA sheets with no fine details. The actual time the EVA slabs are to be set aside for will depend on the size and thickness of the EVA slabs, and the ambient temperature and humidity.
10. Cut the EVA sheets into smaller sizes.
11. Trim off the smooth surface of the EVA sheets and cut the EVA sheets to the desired thickness.

The presence of activatable benign dormant bacteria on the surface of the specimens of the product 10 was tested as follows:
1. Prepare petri dishes containing agar by dispense sterilized tryptic soy broth agar (cooled to 47°C ± 2°C) by pouring 15 ± 2 ml into each standard (15 x 100 mm) flat bottomed petri dish. Allow the agar to gel firmly.
2. Cut test specimens (non-sterile) of the product 10 by hand using sterile scissors, or with a sterile die, into approximately 1 inch x 1 inch squares.
3. For each test specimen press the test specimen into a petri dish to ensure intimate contact of the test specimens with the agar surface.
4. Incubate the petri dishes containing the test specimens for 24 hours at 35°C.
5. Remove the petri dishes containing the test specimens from the incubator.
6. Visually inspect each petri dish to determine the presence or absence of bacterial growth from each test specimen.

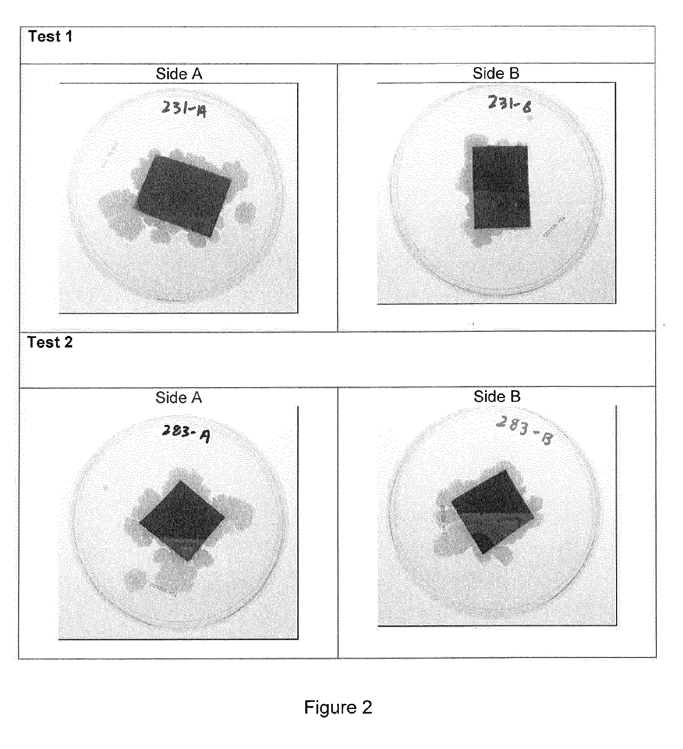

Figure 2 shows representative test results for two test specimens. The test results show good growth the bacteria in the petri dishes radiating outwardly from the test specimens, confirming that the benign dormant bacteria were present on the surface of the test specimens and activatable upon exposure to organic matter.

The surface population of the benign dormant bacteria in the test specimens of the product 10 was estimated as follows:
1. Prepare 500 ml of tryptic soy broth (TSB) per label directions.
2. Add the 500 ml of TSB to a clean 1-liter baffle flask.
3. Add a stopper and foil covering to the 1-liter baffle flask.
4. Repeat steps 1 to 3 to prepare a second 1-litre baffle flask.
5. Autoclave the 1-liter baffle flasks containing the 500 ml of TSB for 15 minutes at 121°C and 15 psi.
6. Autoclave a 12" X 18" paper wrapped stainless steel plate.
7. Allow the stainless steel plate to cool completely.
8. Cool the 1-litre baffle flasks containing TSB to 80°C in an 80°C water bath.
9. Unwrap the stainless steel plate, and place the plate into a biosafety hood.
10. Place a test specimen on top of the stainless steel plate, so that it is laid out flat on the surface of the stainless steel plate.
11. Using a new, disposable scalpel blade attached to a reusable scalpel handle, cut two one-inch squares from the center of the test specimen.
12. Place each one-inch square into one of the sterile 1-liter baffle flasks containing the 500 ml of TSB.
13. Keep the 1-liter baffle flasks in the water bath for 10 minutes.
14. Remove the 1-liter baffle flasks from the water bath and cool to 30°C in a temperature controlled shaker set at 150 RPM.
15. When the 1-liter baffle flasks have cooled to 30°C, begin timing a 24 hour growth period.
16. At the end of the 24-hour growth period, aseptically transfer 10 ml of the TSB broth from each 1-liter baffle flask to a 90 ml buffer dilution
17. 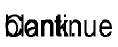 making serial dilutions by adding 10 ml to 90 ml blanks through to a 10⁻⁶ dilution.
18. Plate the 10⁻⁴ through the 10⁻⁶ dilutions onto tryptic soy agar plates.
19. Incubate the tryptic soy agar plates for 24 hours at 35°C.
20. Count the bacterial colonies on each plate.
21. Determine *Bacillus* colony forming units (cfu) per ml from the serial plate count calculations.
22. Divide the serial plate count calculation by 2, to obtain the cfu/ml is the potential for growth from each one-square inch of the test material.

The results ranged between 1.11x10⁹ and 1.55x10⁹ potential colony forming units of growth per one square inch of test specimen. This test confirmed that the desired number of benign dormant bacteria was exposed on the surfaces of the test specimens of the product 10.

When the surfaces of the test specimens were roughed up by scraping with a piece of plastic, the results ranged between 0.92x10⁹ and 1.33x10⁹ potential colony forming units of growth per one square inch of test specimens. This test confirmed that abrasive wear of the surface of the product 10 exposed sufficient fresh benign dormant bacteria buried in the body to the surface for additional odour control.

The present invention is further described by means of the examples, presented below. The use of such examples is illustrative only and in no way limits the scope and meaning of the invention or of any exemplified term, nor to any particular preferred embodiments described herein.

Indeed, many modifications and variations of the invention will be apparent to those skilled in the art upon reading this specification. The invention is therefore to be limited only by the terms of the appended claims.

### EXAMPLE 1: Pressed EVA Footbed for Sandal with Benign Dormant Bacteria Embedded Generally Throughout

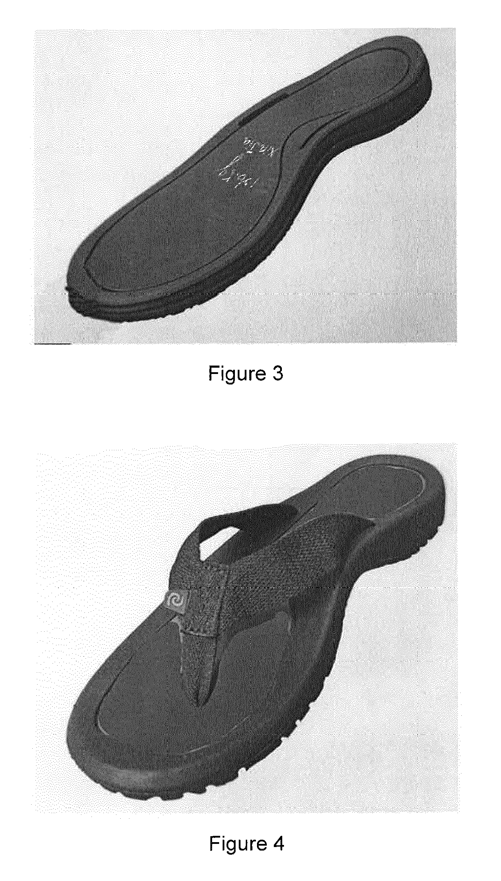

The following example describes a method for making a pressed EVA for a sandal, as shown in Figure 3. In this example, a sample of an aqueous dormant bacteria mixture was added to EVA and melt-blended as follows.

| **INGREDIENTS** | **WEIGHT** | | **SUPPLIER** |
|---|---|---|---|
| | **grams** | **% w/w** | |
| aqueous dormant bacteria mixture (containing about 2.12x10 cfu/ml of *Bacillus megaterium, Bacillus subtilus,* and *Licheniformis* in a ratio of about 1:3:6) | 150 | 0.94 | Envera LLC, West Chester, Pennsylvania |
| EVA resin | 4,086 | 25.58 | The Polyolefin Company (Singapore) Pte. Ltd, Singapore; Formosa Plastics Corporation, Taiwan; Braskem, Brazil; Asia Polymer Corporation, Taiwan |
| CaCO₃ (filler) | 4,540 | 28.42 | Shangdong Bo-Liao, China |
| dicumyl peroxide (DCP) cross-linking agent | 120 | 0.75 | Akzo Nobel Cross-linking Peroxides (Ningbo) CO., Ltd., China; Sinopec, China |
| AC foaming/blowing agent | 450 | 2.82 | |
| zinc oxide (ZnO) blowing agent promoter | 100 | 0.63 | Shijiazhuang Zinc Industry Co., Ltd., China; Taiyang, China; Shijiazhuang Zinc Industry Co., Ltd., China |
| zinc salt of stearic acid (ZnSt) blowing agent promoter | 35 | 0.22 | Pt. Sumi Asih Oleochemicals Industry, Indonesia |
| Polyethylene (PE) | 1,135 | 7.10 | National Petrochemical Company, Thailand; PTT Polyethylene Company Limited, Thailand; Kunlun, China |
| Polyolefin Elastomer (POE) | 2,951 | 18.47 | LG Chem, Korea |
| natural rubber | 908 | 5.68 | |
| color dye - black | 1,500 | 9.39 | |
| **Total** | **15,975 g** | **100 %** | |

### Preparing / Mixing EVA Mixture

1. Add the EVA resin, the CaCO₃, the natural rubber, the ZnO, the ZnSt, the PE, and the POE to a mixing machine set to a temperature of between 115°C and 120°C. Mix the mixture for 5 minutes.
2. Add the dicumyl peroxide (DCP) cross-linking agent, the AC foaming/blowing agent, and the color dye to the mixing machine. Continue mixing the mixture for another 5 minutes.
3. Transfer the mixture to a double-roller blending/kneading machine with 21 inch diameter rollers set to a temperature of 110°C. Knead the mixture for about 2 minutes.
4. Add the aqueous dormant bacteria mixture by slowly pouring it onto the mixture kneading on the roller.
5. Knead the mixture for 3 to 6 minutes.
6. Transfer the mixture to a cutting machine to cut the mixture into thin sheets which are 1.2 mm thick.

### Foaming / Blowing

7. Put stacks of the thin sheets about 16 mm high into a mold of 150 cm 75 cm x 1.6 cm. Insert the mold into a blowing machine at 171°C and apply a pressure of 183 kg/cm² for 22 minutes.
8. Release the pressure and remove the blown EVA slabs, which become about 32 mm thick, from the blowing machine.
9. Set the blown EVA slabs aside for a minimum of 2 hours to allow them to cool and shrink to a stable size, resulting in smooth EVA sheets with no fine details. The actual time the EVA slabs are to be set aside for will depend on the size and thickness of the EVA slabs, and the ambient temperature and humidity.

### Cutting

10. Cut the EVA sheets to smaller sizes.
11. Trim off the smooth surface of the EVA sheets and cut the EVA sheets to the desired thickness.
12. Cut the EVA sheets through a roller with emboss for a design, resulting in a final, detailed sandal footbed, which is then ready for assembly into a sandal, as shown in Figure 4.

### EXAMPLE 2: EVA Footbed for Sandal with Benign Dormant Bacteria Embedded Generally Throughout

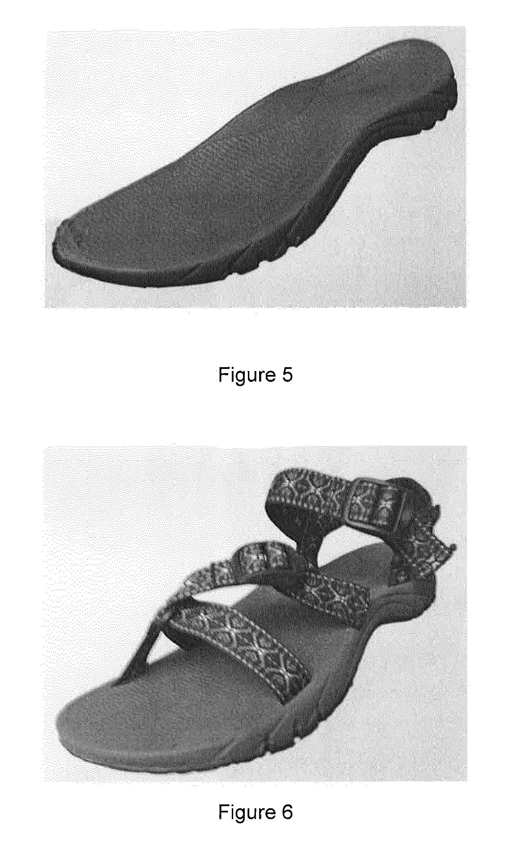

The following example describes a method for making an EVA footbed for a sandal, as shown in Figure 5. The EVA footbeds according to this embodiment provide two layers: a top layer EVA, which the wearer's foot touches, contains dormant bacteria, and a bottom layer that has no dormant bacteria. In this example, a sample of an aqueous dormant bacteria mixture was added to EVA and melt-blended as follows.

| **INGREDIENTS** | **WEIGHT** | | **SUPPLIER** |
|---|---|---|---|
| | **grams** | **% w/w** | |
| aqueous dormant bacteria mixture (containing about 2.12x10 cfu/ml of *Bacillus megaterium, Bacillus subtilus,* and *Licheniformis* in a ratio of about 1:3:6) | 90 | 0.99 | Envera LLC, West Chester, Pennsylvania |
| EVA resin | 6,300 | 69.31 | The Polyolefin Company (Singapore) Pte. Ltd, Singapore; Formosa Plastics Corporation, Taiwan; Braskem, Brazil; Asia Polymer Corporation, Taiwan |
| CaCO₃ (filler) | 1,440 | 15.84 | Shangdong Bo-Liao, China |
| dicumyl peroxide (DCP) cross-linking agent | 45 | 0.50 | Akzo Nobel Cross-linking Peroxides (Ningbo) CO., Ltd., China; Sinopec, China |
| AC foaming/blowing agent | 135 | 1.49 | |
| Zinc Oxide (ZnO) blowing agent promoter | 90 | 0.99 | Shijiazhuang Zinc Industry Co., Ltd., China; Taiyang, China; Shijiazhuang Zinc Industry Co., Ltd., China |
| Zinc Salt of Stearic Acid (ZnSt) blowing agent promoter | 45 | 0.50 | Pt. Sumi Asih Oleochemicals Industry, Indonesia |
| flow promoting agent | 450 | 4.95 | |
| color dye - black | 495 | 5.45 | |
| **Total** | **9,090 g** | **100 %** | |

### Preparing / Mixing EVA Mixture

1. Add the aqueous dormant bacteria mixture to the CaCO₃ filler.
2. Add the EVA resin, the CaCO₃ and dormant bacteria mixture, the ZnO, the ZnSt, and the color dye to a blending/kneading machine set to an initial temperature of 90°C and gradually rising at a rate of about 1.5°C/min. Knead for about 6 minutes.
3. Add the dicumyl peroxide (DCP) cross-linking agent, the AC-foaming agent, and the flow promoting agent to the blending/kneading machine and continue kneading for another 6 minutes, with the temperature of the blending/kneading machine continuing to gradually rise at a rate of 1.5°C/min to 108°C.
4. Transfer the mixture to a pellet-making machine set to a temperature of 90°C, in which the material is cut to round EVA flat pellets measuring 4 mm in diameter and 1-3 mm thick.

### Initial Foaming / Molding to rough EVA Footbed

5. Put the pellets into molds for foaming at 165°C, pressed at 150 kg/cm² for 15 minutes for Men's and 13 minutes for Women's, according to the following weights:
a. for men's size 10, total 140 g:

| | | |
|---|---|---|
| top layer | - | 87 g EVA pellets with benign dormant bacteria |
| bottom layer | - | 53 g EVA pellets without benign dormant Bacteria |

b. for women's size 8, total 99 g:

| | | |
|---|---|---|
| top layer | - | 68 g EVA pellets with benign dormant bacteria |
| bottom layer | - | 31 g EVA pellets without benign dormant bacteria |

6. Remove the rough EVA footbeds from the molds and allow them to cool. The rough EVA footbeds are smooth, slightly larger, general shaped footbeds, with no fine details.
7. Buff the surfaces of the rough EVA footbeds.

### Final Shaping

8. Mold the rough EVA footbeds at a hot pressing temperature of 165°C and a pressing pressure of 80 kg/cm² for 390 seconds for men's and 360 seconds for women's.
9. Cold shape the footbeds at 40°C for 460 seconds.
10. Trim the final, detailed footbeds, which are then ready for assembly into sandals, an example of which is shown in Figure 6.

### EXAMPLE 3: EVA Footbed for Sandal with Benign Dormant Bacteria Embedded Generally Throughout

The following example describes an alternate method for making an EVA footbed for a sandal, as shown in Figure 5. In this example, a sample of an aqueous dormant bacteria mixture was added to EVA and melt-blended as follows.

| **INGREDIENTS** | **WEIGHT** | | **SUPPLIER** |
|---|---|---|---|
| | **grams** | **% w/w** | |
| aqueous dormant bacteria mixture (containing about 2.12x10 cfu/ml of *Bacillus megaterium, Bacillus subtilus,* and *Licheniformis* in a ratio of about 1:3:6) | 33 | 1.01 | Envera LLC, West Chester, Pennsylvania |
| EVA resin | 2,280 | 69.47 | The Polyolefin Company (Singapore) Pte. Ltd, Singapore; |
| CaCO₃ (filler) | 600 | 18.28 | Shangdong Bo-Liao, China |
| dicumyl peroxide (DCP) cross-linking agent | 15 | 0.46 | Akzo Nobel Cross-linking Peroxides (Ningbo) CO., Ltd., China; Sinopec, China |
| AC foaming/blowing agent | 84 | 2.56 | |
| Zinc Oxide (ZnO) blowing agent promoter | 30 | 0.91 | Shijiazhuang Zinc Industry Co., Ltd., China; Taiyang, China; Shijiazhuang Zinc Industry Co., Ltd., China |
| Zinc Salt of Stearic Acid (ZnSt) blowing agent promoter | 15 | 0.46 | Pt. Sumi Asih Oleochemicals Industry, Indonesia |
| flow promoting agent | 18 | 0.55 | |
| color dye - black | 180 | 5.48 | |
| plasticizer | 27 | 0.82 | |
| **Total** | **3,249 g** | **100 %** | |

### Preparing / Mixing EVA Mixture

1. Add the EVA resin, the CaCO₃, the ZnO, and the ZnSt into a mixer set to a temperature of between 105°C and 110°C.
2. Mix the mixture for about 12 minutes.
3. Remove the mixture from the mixer and roll it into 40 cm x 60 cm x 2 mm slabs.
4. Transfer 2,925 g of the slabs to a double-roller blending/kneading machine with 40 cm diameter rollers set to a temperature of 90°C. Knead for about 2 minutes.
5. Add the aqueous dormant bacteria mixture to the blending/kneading machine. Knead for 2 minutes.
6. Add the flow promoting agent, the color dye-black, and the plasticizer (preferably at the same time by pouring out of a pre-mixed bag). Knead for 2 minutes.
7. Add the dicumyl peroxide (DCP) cross-linking agent, and the AC foaming/blowing agent (preferably at the same time by pouring out of a pre-mixed bag). Knead for 6 minutes.
8. Remove the mixture from the blending/kneading machine and roll it out into one big flat sheet about 3 mm thick. Set aside the sheet and allow it to cool down.
9. Cut the sheet into small pellets measuring 3 mm x 6 mm x 9 mm.

### Initial Foaming / Molding to rough EVA Footbed

10. Put the pellets into molds for foaming at 160°C, pressed at 180-200 kg/cm² for 680 seconds, according to the following weights:
   a. for men's size 10: 98.5 g
   b. for women's size 8: 80 g.
11. Remove the rough EVA footbeds from the molds and allow them to cool. The rough EVA footbeds are smooth, slightly larger, general shaped footbeds, with no fine details.
12. Buff the surfaces of the rough EVA footbeds.

### Final Shaping

13. Mold the rough EVA footbeds at a hot pressing temperature of 150°C and a pressing pressure of 55 kg/cm² for 460 seconds.
14. Cold shape the foot beds at 40°C for 460 seconds.
15. Trim the final, detailed footbeds, which are then ready for assembly into a sandal, an example of which is shown in Figure 6.

### EXAMPLE 4: EVA Insole with Benign Dormant Bacteria Embedded Generally Throughout

The following example describes a method for making an EVA insole for a shoe. In this example, an aqueous dormant bacteria mixture was added to EVA and melt-blended as follows.

| **INGREDIENTS** | **WEIGHT** | |
|---|---|---|
| | **grams** | **% w/w** |
| aqueous dormant bacteria mixture (containing about 2.12x10⁹ cfu/ml of *Bacillus megaterium, Bacillus subtilus,* and *Licheniformis* in a ratio of about 1:3:6) obtained from Envera LLC, West Chester, Pennsylvania | 28 | 0.99 |
| EVA resin | 1,000 | 35.38 |
| dicumyl peroxide (DCP) cross-linking agent | 51.18 | 1.81 |
| AC foaming/blowing agent | 110.27 | 3.90 |
| zinc oxide (ZnO) blowing agent promoter | 50 | 1.77 |
| zinc salt of stearic acid (ZnSt) blowing agent promoter | 25 | 0.88 |
| polyethylene (PE) or polyolefin elastomer (POE) | 1,500 | 53.08 |
| color dye - black | 41.61 | 1.47 |
| powder (Tyressen Co., Ltd., 426-19 Yeocheon Dong, Nam-gu, Ulsan, South Korea) | 20.06 | 0.71 |
| **Total** | **2,826.12 g** | **100 %** |

### Preparing / Mixing EVA Mixture

1. Mix the ZnO and ZnSt powders in a container and add the aqueous dormant bacteria mixture.
2. Add the EVA resin, and the PE or POE into a mixer set to a temperature of 80°C, and a mixing speed of 500 to 580 rpm.
3. Mix the mixture for about 1 minute.
4. Add the mixture of ZnO, ZnSt, and dormant bacteria mixture from step 1 above to the mixer. Mix the mixture at 90°C and a mixing speed of 580 to 740 rpm, for 10 minutes.

### Kneading

5. Transfer the mixture from step 4 to a double-roller blending/kneading machine with 21 inch diameter rollers set to a temperature of 100°C. Knead for about 5 minutes.
6. Add the AC foaming agent and the color dye to the mixture. Continue mixing the mixture for another 3.5 minutes.
7. Add the dicumyl peroxide (DCP) cross-linking agent, and the powder to the mixture. Continue mixing the mixture for another 5 minutes.

### Blowing / Foaming

8. Transfer a portion of the mixture from step 7 into a mold measuring 17.5 cm x 12 cm x 2.3 cm.
9. Insert the mold into a blowing machine at 160°C and apply a pressure of 155 kg/cm² for 35 minutes.
10. Release the pressure and take the blown EVA slab from the mold.
11. Set the blown EVA slab aside to cool and shrink to its stable size. Results in a smooth EVA sheet, with no fine details.
12. Repeat steps 8-11 above until the entire mixture is foamed.

### Cutting

13. Cut the EVA sheets into smaller sizes.
14. Trim off the smooth surface of the EVA sheets and cut the EVA sheets to the desired thickness.
15. Cut insoles out from the EVA sheets.

While reference has been made to various preferred embodiments of the invention other variations, implementations, modifications, alterations and embodiments are comprehended by the broad scope of the appended claims. Some of these have been discussed in detail in this specification and others will be apparent to those skilled in the art. Those of ordinary skill in the art having access to the teachings herein will recognize these additional variations, implementations, modifications, alterations and embodiments, all of which are within the scope of the present invention, which invention is limited only by the appended claims.

### THE INVENTION WILL NOW BE DESCRIBED BY REFERENCE TO THE FOLLOWING CLAUSES:

1. A product capable of controlling odours arising from organic matter, said product comprising:
   an admixture of a moldable thermoplastic and benign dormant bacteria in a sporulated form, formed by admixing said benign dormant bacteria and said moldable thermoplastic when said moldable thermoplastic is melted, said admixture being molded into a molded product;
   said benign dormant bacteria being embedded throughout said molded product, including being buried within a body of said molded product and being presented on a surface of said molded product, said benign dormant bacteria presented on said surface being activatable upon exposure of said surface to said organic matter;
   wherein said benign activated bacteria digest said organic matter to control production of offensive odours, and abrasive wear of said surface exposes fresh benign dormant bacteria buried within said body to said surface for additional odour control.
2. The product according to clause 1, wherein said molded product contains a surface population of said benign dormant bacteria large enough to digest said organic matter at a rate sufficient to control said odours.
3. The product according to clause 2, wherein between 10⁶ and 10⁸ of said benign dormant bacteria are present on said surface per square inch of said surface.
4. The product according to clause 1, wherein said molded product contains a buried population of said benign bacteria large enough to continue to digest said organic matter at a rate sufficient to control said odours as said surface wears.
5. The product according to clause 4, wherein 10¹² to 10¹⁶ of said benign dormant bacteria are present in said molded product per cubic inch of said molded product.
6. The product according to clause 1, wherein said moldable thermoplastic comprises an ethylene vinyl acetate (EVA), a polyethylene, a polypropylene, a polyvinyl chloride, a polyamide, an elastane, or a polystyrene.
7. The product according to clause 6, wherein said EVA is an open-cell foam, or a closed-cell foam.
8. The product according to clause 1, having a compression molded shape, an injection molded shape, a blow molded shape, or an extrusion molded shape.
9. The product according to clause 1, wherein said organic matter is a bodily fluid, or organic waste, containing carbon, nitrogen, and sufficient moisture content to activate said benign dormant bacteria.
10. The product according to clause 9, wherein said bodily fluid is sweat, blood, saliva, or semen.
11. The product according to clause 9, wherein said organic waste is feces, urine, milk, wine, a soft drink, syrup, or an egg.
12. The product according to clause 1, wherein said dormant bacteria comprises at least one strain of the genus *Bacillus.*
13. The product according to clause 12, wherein said at least one strain of the genus *Bacillus,* when activated, produces an enzyme to break down and assist with said digesting said organic matter.
14. The product according to clause 13, wherein said at least one strain of the genus *Bacillus* produces water and carbon dioxide as by-products of said break down and said digesting said organic matter.
15. The product according to clause 13, wherein said at least one strain of the genus *Bacillus* returns to a dormant state once said organic matter has been digested.
16. The product according to clause 12, wherein said at least one strain of the genus *Bacillus* is selected from the group consisting of *Bacillus megaterium, Bacillus subtilus, Bacillus Licheniformis, Bacillus Cerus, Bacillus, Alvei, Bacillus Coagulans, Bacillus Pumillus, Bacillus Simplex, Bacillus Brevis,* and *Bacillus Amyloliquefaciens.*
17. The product according to clause 16, wherein said dormant bacteria comprises strains of *Bacillus megaterium, Bacillus subtilus,* and *Bacillus Licheniformis.*
18. The product according to clause 1, in the form of a mat, or at least a portion of a footwear.
19. The product according to clause 18, wherein said mat is a sports mat, a gym mat, an exercise mat, a yoga mat, a child's play mat, or a garden mat.
20. The product according to clause 18, wherein said at least portion of footwear is a sandal, a shoe, a boot, a flip flop, a slipper, a clog, an insole, a footbed, or an outsole.
21. The product according to clause 1, in the form of a fiber, a pellet, a sheet, a block, or a tile.
22. A secondary structure formed by cutting, abrading, shaping, forming, or additional molding, said sheet, block, or tile form of clause 21.
23. A secondary structure formed by braiding, twisting, weaving, knitting, crocheting, knotting, spinning, crimping, or felting, said fiber of clause 21.
24. The secondary structure as described in clause 23, in the form of a thread, a yarn, or a textile.
25. The secondary structure as described in clause 24, wherein said textile is a floor covering, bedding, drapery, upholstery, a towel, at least a portion of an article of clothing, or at least a portion of a footwear.
26. The secondary structure as described in clause 25, wherein said floor covering is a carpet, a carpet tile, a mat, or a rug.
27. The secondary structure as described in clause 25, wherein said article of clothing is a dress, a coat, a sock, pants, a shirt, headwear, or a jacket.
28. The secondary structure as described in clause 25, wherein said at least portion of said footwear is a sandal, a shoe, a boot, a slipper, a flip flop, a clog, an insole, a footbed, or an outsole.
29. A method of making a product capable of controlling odours arising from organic matter, said method comprising the steps of:
   a) melt-blending at a bacteria compatible temperature range:
      i) a moldable thermoplastic; and
      ii) benign dormant bacteria in a sporulated form; and
   b) forming the melt-blended product of step (a) into a molded product, with said benign dormant bacteria being embedded throughout said molded product, including being buried within a body of said molded product and being presented on a surface of said molded product, said benign dormant bacteria presented on said surface being activatable upon exposure of said surface to said organic matter;
      wherein said benign activated bacteria digest said organic matter to control production of offensive odours, and wherein abrasive wear of said surface exposes fresh benign dormant bacteria buried within said body to said surface for additional odour control.
30. The method as described in clause 29, wherein said molded product contains a surface population of said benign dormant bacteria large enough to digest said organic matter at a rate sufficient to control said odours.
31. The method as described in clause 30, wherein 10⁶ to 10⁸ of said benign dormant bacteria are present on said surface per square inch of said surface.
32. The method as described in clause 31, wherein said molded product contains a buried population of benign bacteria large enough to continue to digest said organic matter at a rate sufficient to control said odours as said surface wears.
33. The method as described in clause 32, wherein 10¹² to 10¹⁶ of said benign dormant bacteria are present in said molded product per cubic inch of said molded product.
34. The method as described in clause 29, wherein the ratio of said benign dormant bacteria to said moldable thermoplastic is about 1:99 by weight.
35. The method as described in clause 29, wherein said moldable thermoplastic comprises one or more of a foaming agent, a cross-linking agent, a lubricant, and a filler.
36. The method as described in clause 29, wherein said moldable thermoplastic comprises an ethylene vinyl acetate (EVA), a polyethylene, a polypropylene, a polyvinyl chloride, a polyamide, an elastane, or a polystyrene.
37. The method as desribed in clause 36, wherein said EVA is an open-cell, or a closed-cell foam.
38. The method as described in clause 29, wherein said benign dormant bacteria comprises an aqueous carrier.
39. The method as described in clause 38, wherein said aqueous carrier comprises water.
40. The method as described in clause 39, wherein said aqueous carrier further comprises a preservative to prevent activation of said benign dormant bacteria.
41. The method as described in clause 40, wherein said preservative is a biocide.
42. The method as described in clause 29, wherein said benign dormant bacteria comprises a dry powder carrier.
43. The method as described in clause 42, wherein said dry powder carrier comprises calcium carbonate, or sodium bicarbonate.
44. The method as described in clause 38 or 42, wherein the concentration of said benign dormant bacteria in said carrier is about 0.0001 % to 2% by weight.
45. The method as described in clause 29, wherein said benign dormant bacteria comprises at least one strain of the genus *Bacillus.*
46. The method as described in clause 45, wherein said at least one strain of the genus *Bacillus,* when activated, produces enzymes to break down and assist with said digesting said organic matter.
47. The method as described in clause 46, wherein said at least one strain of the genus *Bacillus* produces water and carbon dioxide as by-products of said break down and said digesting said organic matter.
48. The method as described in clause 46, wherein said at least one strain of the genus *Bacillus* returns to a dormant state once said organic matter has been digested.
49. The method as described in clause 45, wherein said at least one of the genus *Bacillus* is selected from the group consisting of *Bacillus megaterium, Bacillus subtilus, Bacillus Licheniformis, Bacillus Cerus, Alvei, Bacillus Coagulans, Bacillus Pumillus, Bacillus Simplex, Bacillus* and *Bacillus Amyloliquefaciens.*
50. The method as described in clause 49, wherein said dormant bacteria comprises strains of *Bacillus megaterium, Bacillus subtilus,* and *Bacillus Licheniformis.*
51. The method as described in clause 50, wherein the ratio of said *Bacillus megaterium:Bacillus subtilus:Licheniformis* is about 1:3:6 by weight.
52. The method as described in clause 29, wherein said organic matter is a bodily fluid, or organic waste, containing carbon, nitrogen, and sufficient moisture content to activate said benign dormant bacteria.
53. The method as described in clause 52, wherein said bodily fluid is sweat, blood, saliva, or semen.
54. The method as described in clause 52, wherein said organic waste is feces, urine, milk, wine, a soft drink, syrup, or an egg.
55. The method as described in clause 29, wherein said product is at least a portion of a mat, or at least a portion of a footwear.
56. The method as described in clause 55, wherein said mat is a sports mat, a gym mat, an exercise mat, a yoga mat, or a garden mat.
57. The method as described in clause 55, wherein said footwear is a sandal, a shoe, a boot, a flip flop, a slipper, or a clog.
58. The method as described in clause 29, wherein said product is a fiber, pellet, a sheet, a block, or a tile.
59. The method as described in clause 58, further comprising cutting, abrading, shaping, forming, or additional molding, said sheet, block, or tile into a secondary structure.
60. The method as described in clause 58, further comprising braiding, twisting, weaving, knitting, crocheting, knotting, spinning, crimping, or felting, said fiber into a secondary structure.
61. The method as described in clause 60, wherein said secondary structure is a thread, a yarn, or a textile.
62. The method as described in clause 61, wherein said textile is a floor covering, bedding, drapery, upholstery, a towel, at least a portion of an article of clothing, or at least a portion of a footwear.
63. The method as described in clause 62, wherein said floor covering is a carpet, a carpet tile, a mat, or a rug.
64. The method as described in clause 63, wherein said mat is a sports mat, a gym mat, an exercise mat, yoga mat, or a garden mat.
65. The method as described in clause 62, wherein said article of clothing is a dress, a coat, a sock, pants, a shirt, a jacket, or headwear.
66. The method as described in clause 62, wherein said footwear is a sandal, a shoe, a boot, a flip flop, a slipper, or a clog.
67. The method as described in clause 29, wherein said forming said melt-blended product step comprises the steps of:
   kneading said melt-blended product at a temperature of about 20°C to about 25°C for about 10 minutes;
   rolling said melt-blend product into a sheet;
   compressing said sheet;
   heating said sheet to about 170°C to about 175°C, at a pressure of about 150 kg/cm², for about 10 to 20 minutes, to foam said sheet;
   cross-linking said sheet; and
   compression molding said sheet at about 160°C for about 15 minutes.
68. The method as described in clause 67, wherein said rolling said melt-blended product step further comprises cutting said sheet into smaller sheets.
69. The method as described in clause 29, wherein said forming said melt-blended product step comprises injection molding, blow molding, or extrusion molding, said melt-blend product.
70. The method as described in clause 69, further comprising compression molding said injection molded, blow molded, or extrusion molded product
71. A product capable of controlling odours arising from organic matter, said product being formed by the method as described in any one of clauses 29 to 70.
72. A use of benign dormant bacteria in a sporulated form in the preparation of a product capable of controlling odours arising from organic matter, said product comprising an admixture of a moldable thermoplastic benign dormant bacteria in a sporulated form, formed by admixing said dormant bacteria and said moldable thermoplastic when said moldable thermoplastic is melted, said admixture being molded into a molded product, said benign dormant bacteria being embedded throughout said molded product, including being buried within a body of said molded product and presented on a surface of said molded product, said benign dormant bacteria presented on said surface being activatable upon exposure of said surface to said organic matter, wherein said benign activated bacteria digest said matter to control production of offensive odours, and abrasive wear of said surface exposes fresh benign dormant bacteria buried within said body to surface for additional odour control.
73. The use as described in clause 72, wherein said molded product contains a surface population of said benign dormant bacteria large enough to digest said organic matter at a rate sufficient to control said odours.
74. The use as described in clause 73, wherein between 10⁶ and 10⁸ benign dormant bacteria are present on said surface per square inch of said surface.
75. The use as described in clause 72, wherein said molded product contains a buried population of benign bacteria large enough to continue to digest said organic matter at a rate sufficient to control said odours as said surface wears.
76. The use as described in clause 75, wherein between 10¹² and 10¹⁶ benign dormant bacteria are present in said molded product per cubic inch of said molded product.
77. The use as described in clause 72, wherein the ratio of said benign dormant bacteria to said moldable thermoplastic is about 1:99 by weight.
78. The use as described in clause 72, wherein said moldable thermoplastic comprises an ethylene vinyl acetate (EVA), a polyethylene, a polypropylene, a polyvinyl chloride, a polyamide, an elastane, or a polystyrene.
79. The use as described in clause 78, wherein said moldable thermoplastic further comprises one or more of a foaming agent, a cross-linking agent, a lubricant, and a filler.
80. The use as described in clause 78, wherein said EVA is an open-cell, or a closed-cell foam.
81. The use as described in clause 72, wherein said preparation involves melt-blending at a bacteria compatible temperature range said moldable thermoplastic and said benign dormant bacteria to form a melt-blended product.
82. The use as described in clause 81, wherein said preparation further involves injection molding, blow molding, or extrusion molding, said melt-blended product.
83. The use as described in clause 82, further comprising compression molding said injection molded, blow molded, or extrusion molded product
84. The use as described in clause 72, wherein said benign dormant bacteria comprises an aqueous carrier.
85. The use as described in clause 84, wherein said aqueous carrier comprises water.
86. The use as described in clause 85, wherein said aqueous carrier comprises a preservative to prevent activation of said benign dormant bacteria.
87. The use as described in clause 86, wherein said preservative is a biocide.
88. The use as described in clause 72, wherein said benign dormant bacteria comprises a dry powder carrier.
89. The use as described in clause 88, wherein said dry powder carrier comprises calcium carbonate, or sodium bicarbonate.
90. The use as described in clause 84 or 88, wherein the concentration of said benign dormant bacteria in said carrier is about 0.0001% to 2% by weight.
91. The use as described in clause 72, wherein said benign dormant bacteria comprises at least one strain of the genus *Bacillus.*
92. The use as described in clause 91, wherein said at least one strain of the genus *Bacillus,* when activated, produces enzymes to break down and assist with said digesting said organic matter.
94. The use as described in clause 92, wherein said at least one strain of the genus *Bacillus* produces water and carbon dioxide as by-products of said break down and said digesting said organic matter.
95. The use as described in clause 92, wherein said at least one strain of the genus *Bacillus* returns to a dormant state once said organic matter has been digested.
96. The use as described in clause 91, wherein said at least one strain of the genus *Bacillus* is selected from the group consisting of *Bacillus megaterium, Bacillus subtilus, Bacillus Licheniformis, Bacillus Cerus, Bacillus, Alvei, Bacillus Coagulans, Bacillus Pumillus, Bacillus Simplex, Bacillus Brevis,* and *Bacillus Amyloliquefaciens.*
97. The use as described in clause 96, wherein said dormant bacteria comprises strains of *Bacillus megaterium, Bacillus subtilus,* and *Bacillus Licheniformis.*
98. The method as described in clause 97, wherein the ratio of said *Bacillus megaterium:Bacillus subtilus:Licheniformis* is about 1:3:6 by weight.
99. The use as described in clause 72, wherein said organic matter is a bodily fluid, or organic waste, containing carbon, nitrogen, and sufficient moisture content to activate said benign dormant bacteria.
100. The use as described in clause 99, wherein said bodily fluid is sweat, blood, saliva, or semen.
101. The use as described in clause 99, wherein said organic waste is feces, urine, milk, wine, a soft drink, syrup, or an egg.
102. The use as described in clause 72, wherein said molded product is a mat, or at least a portion of a footwear.
103. The use as described in clause 102, wherein said mat is a sports mat, a gym mat, an exercise mat, a yoga mat, or a garden mat.
104. The use as described in clause 102, wherein said at least portion of said footwear is a sandal, a shoe, a boot, a flip flop, a slipper, a clog, an a footbed, or an outsole.
105. The use as described in clause 72, wherein said molded product is a fiber, a pellet, a sheet, a block, or a tile.
106. The use as described in clause 105, wherein said preparation further comprises cutting, abrading, shaping, forming, or additional molding, said sheet, block, or tile into a secondary structure.
107. The use as described in clause 105, wherein said preparation further comprises braiding, twisting, weaving, knitting, crocheting, knotting, spinning, crimping, or felting, said fiber into a secondary structure.
108. The use as described in clause 107, wherein said secondary structure is a thread, a yarn, or a textile.
109. The use as described in clause 108, wherein said textile is a floor covering, bedding, drapery, upholstery, a towel, at least a portion of an article of clothing, or at least a portion of a footwear.
110. The use as described in clause 109, wherein said floor covering is a carpet, a carpet tile, a mat, or a rug.
111. The use as described in clause 109, wherein said article of clothing is a dress, a coat, a sock, pants, a shirt, a jacket, or headwear.
112. The method as described in clause 109, wherein said at least portion said footwear is a sandal, a shoe, a boot, a flip flop, a slipper, a clog, an a footbed, or an outsole.

### THE INVENTION WILL NOW BE DESCRIBED BY REFERENCE TO THE FOLLOWING FURTHER CLAUSES:

1. A product capable of controlling odours arising from organic matter, said product comprising:
   an admixture of a moldable thermoplastic and benign dormant bacteria in a sporulated form, formed by admixing said benign dormant bacteria and said moldable thermoplastic when said moldable thermoplastic is melted, said admixture being molded into a molded product;
   said benign dormant bacteria being embedded throughout said molded product, including being buried within a body of said molded product and being presented on a surface of said molded product, said benign dormant bacteria presented on said surface being activatable upon exposure of said surface to said organic matter;
   wherein said benign activated bacteria digest said organic matter to control production of offensive odours, and abrasive wear of said surface exposes fresh benign dormant bacteria buried in said body to said surface for additional odour control.
2. The product according to clause 1, wherein at least 10⁶ of said benign dormant bacteria are present per square inch of said surface.
3. The product according to clause 1, wherein at least 10¹² of said benign dormant bacteria are present per cubic inch of said molded product.
4. The product according to clause 2, wherein at least 10¹² of said dormant bacteria are present per cubic inch of said molded product.
5. The product according to clause 1, wherein said moldable thermoplastic comprises ethylene vinyl acetate (EVA), polyethylene, polypropylene, polyvinyl chloride, polyamide, elastane or polystyrene.
6. The product according to clause 5, wherein said thermoplastic is an open-cell foam.
7. The product according to clause 5, wherein said thermoplastic is an closed-cell foam.
8. The product according to clause 6, wherein said thermoplastic is EVA.
9. The product according to clause 7, wherein said thermoplastic is EVA.
10. The product according to clause 1, which is compression molded, injection molded, blow molded or extrusion molded.
11. The product according to clause 1, wherein said dormant bacteria comprises at least one strain of the genus *Bacillus.*
12. The product according to clause 11, wherein said *Bacillus,* when activated, produces an enzyme to break down and assist with said digesting said organic matter.
13. The product according to clause 12, wherein said *Bacillus* produces water and carbon dioxide as by-products of said break down and digesting of said organic matter.
14. The product according to clause 12, wherein said *Bacillus* returns to a dormant state once said organic matter has been digested.
15. The product according to clause 12, wherein said *Bacillus* is selected from the group consisting of *Bacillus megaterium, Bacillus subtilus, Bacillus Licheniformis, Bacillus Cerus, Bacillus, Alvei, Bacillus Coagulans, Bacillus Pumillus, Bacillus Simplex, Bacillus Brevis* and *Bacillus Amyloliquefaciens.*
16. The product according to clause 15, wherein said dormant bacteria comprises *Bacillus megaterium, Bacillus subtilus* and *Bacillus Licheniformis.*
17. The product according to clause 1, in the form of a sports mat.
18. The product according to clause 1, in the form of footwear.
19. The product according to clause 1, in the form of an insole, a footbed or an outsole.
20. The product according to clause 1, in the form of a fiber, a pellet, a sheet, a block or a tile.
21. The product according to clause 1, in the form of a thread or a yarn.
22. The product according to clause 1, in the form of a floor covering, bedding, drapery, upholstery, towel or at least a portion of an article of clothing.
23. The product according to clause 1, in the form of a carpet, carpet tile, mat or rug.
24. The product according to clause 1, wherein said organic matter is a bodily fluid or organic waste, said bodily fluid or organic waste contains sufficient carbon, nitrogen and moisture to activate said benign dormant bacteria.
25. The product according to clause 24, wherein said bodily fluid is feces, urine, sweat, blood, saliva or semen.
26. The product according to clause 24, wherein said organic waste is milk, wine, a soft drink, syrup or an egg.
27. A method of making a product capable of controlling odours arising from organic matter, comprising the steps of:
   a) melt-blending at a bacteria compatible temperature range:
      i) a moldable thermoplastic; and
      ii) benign dormant bacteria in a sporulated form; and
   b) forming the melt-blended product of step (a) into a molded product, with said benign dormant bacteria being embedded throughout said molded product, including being buried within a body of said molded product and being presented on a surface of said molded product, said benign dormant bacteria presented on said surface being activatable upon exposure of said surface to said organic matter;
   wherein said benign activated bacteria digest said organic matter to control production of offensive odours, and abrasive wear of said surface exposes fresh benign dormant bacteria buried within said body to said surface for additional odour control.
28. The method as described in clause 27, wherein at least 10⁶ of said benign dormant bacteria are present per square inch of said surface.
29. The method as described in clause 27, wherein at least 10¹² of said benign dormant bacteria are present per cubic inch of said molded product.
30. The method as described in clause 28, wherein at least 10¹² of said benign dormant bacteria are present per cubic inch of said molded product.
31. The method as described in clause 27, wherein said benign dormant bacteria comprises an aqueous carrier.
32. The method as described in clause 27, wherein said benign dormant bacteria comprises a dry powder carrier.
33. The method as described in clause 32, wherein said dry powder carrier comprises calcium carbonate or sodium bicarbonate.
34. The method as described in clause 31, wherein the concentration of said benign dormant bacteria in said carrier is about 0.0001% to 2% by weight.
35. The method as described in clause 32, wherein the concentration of said benign dormant bacteria in said carrier is about 0.0001% to 2% by weight.
36. The method as described in clause 33, wherein the concentration of said benign dormant bacteria in said carrier is about 0.0001% to 2% by weight.
37. The method as described in clause 27, wherein said benign dormant bacteria comprises at least one strain of the genus *Bacillus.*
38. The method as described in clause 37, wherein said *Bacillus* is selected from the group consisting of *Bacillus megaterium, Bacillus subtilus, Bacillus Licheniformis, Bacillus Cerus, Bacillus, Alvei, Bacillus Coagulans, Bacillus Pumillus, Bacillus Simplex, Bacillus Brevis* and *Bacillus Amyloliquefaciens.*
39. The method as described in clause 38, wherein said dormant bacteria comprises *Bacillus megaterium, Bacillus subtilus* and *Bacillus Licheniformis.*
40. The method as described in clause 27, wherein said forming said melt-blended product step comprises the steps of:
   kneading said melt-blended product at a temperature of about 20°C to about 25°C;
   rolling said melt-blend product into a sheet;
   compressing said sheet;
   heating said sheet to about 170°C to about 175°C, at a pressure of about 150 kg/cm²; and
   compression molding said sheet at about 160°C.
41. The method as described in clause 27, wherein forming said melt-blended product step comprises injection molding, blow molding or extrusion molding.
42. The method as described in clause 41, further comprising compression molding said injection molded, blow molded or extrusion molded product.

## Claims

1. A product capable of controlling odours arising from organic matter,
said product comprising:
an admixture of a moldable thermoplastic and benign dormant bacteria in a sporulated form, formed by admixing said benign dormant bacteria and said moldable thermoplastic when said moldable thermoplastic is melted, said admixture being molded into a molded product;
said benign dormant bacteria being embedded throughout said molded product, including being buried within a body of said molded product and being presented on a surface of said molded product, said benign dormant bacteria presented on said surface being activatable upon exposure of said surface to said organic matter;
wherein said benign activated bacteria digest said organic matter to control production of offensive odours, and abrasive wear of said surface exposes fresh benign dormant bacteria buried within said body to said surface for additional odour control.

2. A method of making a product capable of controlling odours arising from organic matter, said method comprising the steps of:
a) melt-blending at a bacteria compatible temperature range:
i) a moldable thermoplastic; and
ii) benign dormant bacteria in a sporulated form; and
b) forming the melt-blended product of step (a) into a molded product, with said benign dormant bacteria being embedded throughout said molded product, including being buried within a body of said molded product and being presented on a surface of said molded product, said benign dormant bacteria presented on said surface being activatable upon exposure of said surface to said organic matter;
wherein said benign activated bacteria digest said organic matter to control production of offensive odours, and wherein abrasive wear of said surface exposes fresh benign dormant bacteria buried within said body to said surface for additional odour control.

3. A use of benign dormant bacteria in a sporulated form in the preparation of a product capable of controlling odours arising from organic matter, said product comprising an admixture of a moldable thermoplastic and benign dormant bacteria in a sporulated form, formed by admixing said benign dormant bacteria and said moldable thermoplastic when said moldable thermoplastic is melted, said admixture being molded into a molded product, said benign dormant bacteria being embedded throughout said molded product, including being buried within a body of said molded product and being presented on a surface of said molded product, said benign dormant bacteria presented on said surface being activatable upon exposure of said surface to said organic matter, wherein said benign activated bacteria digest said organic matter to control production of offensive odours, and abrasive wear of said surface exposes fresh benign dormant bacteria buried within said body to said surface for additional odour control.

4. The product as claimed in claim 1, in the form of at least a portion of a footwear, or a mat, optionally wherein said at least portion of said footwear is a sandal, a footbed, a shoe, a boot, a flip flop, a slipper, a clog, an insole, or an outsole, or wherein said mat is a sports mat, a gym mat, an exercise mat, a yoga mat, a child's play mat, or a garden mat.

5. The product as claimed in claim 1, the method as claimed in claim 2, or the use as claimed in claim 3, wherein said molded product contains a surface population of said benign dormant bacteria large enough to digest said organic matter at a rate sufficient to control said odours, optionally
wherein between 10⁶ and 10⁸ of said benign dormant bacteria are present on said surface per square inch of said surface, or
wherein said molded product contains a buried population of said benign bacteria large enough to continue to digest said organic matter at a rate sufficient to control said odours as said surface wears, optionally
wherein 10¹² to 10¹⁶ of said benign dormant bacteria are present in said molded product per cubic inch of said molded product.

6. The product as claimed in claim 1, the method as claimed in claim 2, or the use as claimed in claim 3, wherein said moldable thermoplastic comprises an ethylene vinyl acetate (EVA), a polyethylene, a polypropylene, a polyvinyl chloride, a polyamide, an elastane, or a polystyrene, optionally
wherein said EVA is an open-cell foam, or a closed-cell foam, or
having a compression molded shape, an injection molded shape, a blow molded shape, or an extrusion molded shape, or
wherein said organic matter is a bodily fluid, or organic waste, containing carbon, nitrogen, and sufficient moisture content to activate said benign dormant bacteria, optionally
wherein said bodily fluid is sweat, blood, saliva, or semen, or
wherein said organic waste is feces, urine, milk, wine, a soft drink, syrup, or an egg.

7. The product as claimed in claim 1, the method as claimed in claim 2, or the use as claimed in claim 3, wherein said dormant bacteria comprises at least one strain of the genus *Bacillus,* optionally
wherein said at least one strain of the genus *Bacillus,* when activated, produces an enzyme to break down and assist with said digesting said organic matter, optionally
wherein said at least one strain of the genus *Bacillus* produces water and carbon dioxide as by-products of said break down and said digesting said organic matter, or
wherein said at least one strain of the genus *Bacillus* returns to a dormant state once said' organic matter has been digested, optionally
wherein said at least one strain of the genus *Bacillus* is selected from the group consisting of *Bacillus megaterium, Bacillus subtilus, Bacillus Licheniformis, Bacillus Cerus, Bacillus, Alvei, Bacillus Coagulans, Bacillus Pumillus, Bacillus Simplex, Bacillus Brevis,* and *Bacillus Amyloliquefaciens,* preferably
wherein said dormant bacteria comprises strains of *Bacillus megaterium, Bacillus subtilus,* and *Bacillus Licheniformis.*

8. The product as claimed in claim 1, the method as claimed in claim 2, or the use as claimed in claim 3, wherein said product is in the form of a fiber, a pellet, a sheet, a block, or a tile.

9. A secondary structure formed by cutting, abrading, shaping, forming, or additional molding, said sheet, block, or tile form of claim 9, or by braiding, twisting, weaving, knitting, crocheting, knotting, spinning, crimping, or felting, said fiber of claim 21, optionally
in the form of a thread, a yarn, or a textile, optionally
wherein said textile is a floor covering, bedding, drapery, upholstery, a towel, at least a portion of an article of clothing, or at least a portion of a footwear, optionally
wherein said floor covering is a carpet, a carpet tile, a mat, or a rug, or
wherein said article of clothing is a dress, a coat, a sock, pants, a shirt, headwear, or a jacket, or
wherein said at least portion of said footwear is a sandal, a shoe, a boot, a slipper, a flip flop, a clog, an insole, a footbed, or an outsole.

10. The method as claimed in claim 2 or the use as claimed in claim 3, wherein the ratio of said benign dormant bacteria to said moldable thermoplastic is about 1:99 by weight, or wherein said moldable thermoplastic comprises one or more of a foaming agent, a cross-linking agent, a lubricant, and a filler.

11. The method as claimed in claim 2, or the use as claimed in claim 3, wherein said benign dormant bacteria comprises an aqueous carrier, optionally
wherein said aqueous carrier comprises water, optionally
wherein said aqueous carrier further comprises a preservative to prevent activation of said benign dormant bacteria, optionally
wherein said preservative is a biocide, or
wherein said benign dormant bacteria comprises a dry powder carrier, optionally.
wherein said dry powder carrier comprises calcium carbonate, or sodium bicarbonate, optionally wherein the concentration of said benign dormant bacteria in said carrier is about 0.0001% to 2% by weight.

12. The method or use as claimed in claim 10, wherein the ratio of said *Bacillus megaterium:Bacillus subtilus:Licheniformis* is about 1:3:6 by weight.

13. The method as claimed in claim 29, wherein said forming said melt- blended product step comprises the steps of:
kneading said melt-blended product at a temperature of about 20°C to about 25°C for about 10 minutes;
rolling said melt-blend product into a sheet;
compressing said sheet;
heating said sheet to about 170°C to about 175°C, at a pressure of about 150 kg/cm² for about 10 to 20 minutes, to foam said sheet;
cross-linking said sheet; and
compression molding said sheet at about 160°C for about 15 minutes, optionally
wherein said rolling said melt-blended product step further comprises cutting said sheet into smaller sheets.

14. The method as claimed in claim 2, wherein said forming said melt-blended product step comprises injection molding, blow molding, or extrusion molding, said melt-blend product, or the use as claimed in claim 3, wherein said preparation involves melt-blending at a bacteria compatible temperature range said moldable thermoplastic and said benign dormant bacteria to form a melt-blended product, optionally wherein said preparation involves melt-blending at a bacteria compatible temperature range said moldable thermoplastic and said benign dormant bacteria to form a melt-blended product, optionally
further comprising compression molding said injection molded, blow molded, or extrusion molded product.

15. A product capable of controlling odours arising from organic matter, said product being formed by the method according to any preceding claim.
